# FASCICULE DE BREVET EUROPEEN

(11) **EP 3 174 631 B1**
(45) Date de publication et mention de la délivrance du brevet: **07.08.2019**
(21) Numéro de dépôt: 15732582.0
(22) Date de dépôt: 22.06.2015
(51) Int. Cl.: B01D 53/02, C07C 7/12, B01J 20/08, B01J 20/04, B01J 20/32, B01J 20/28, B01J 20/30, B01J 20/34, B01D 53/04

(54) **ADSORBANT A BASE D'ALUMINE CONTENANT DU SODIUM ET DOPEE PAR UN ELEMENT ALCALIN POUR LA CAPTATION DE MOLECULES ACIDES**
ADSORPTIONSMITTEL AUF BASIS VON ALUMINIUMOXID, DAS NATRIUM ENTHÄLT UND MIT EINEM ALKALIELEMENT DOTIERT IST, ZUM EINFANGEN VON SÄUREMOLEKÜLEN
ADSORBENT BASED ON ALUMINA CONTAINING SODIUM AND DOPED WITH AN ALKALINE ELEMENT, FOR THE CAPTURE OF ACID MOLECULES

(30) Priorité: 31.07.2014 FR 1457406
(43) Date de publication de la demande: 07.06.2017
(73) Titulaire: IFP Energies nouvelles, 92500 Rueil-Malmaison (FR)
(72) Inventeur: BARTHELET, Karin, F-69007 Lyon (FR); BAUDOT, Arnaud, F-69390 Vernaison (FR); LELIAS, Marc-Antoine, F-30100 Ales (FR); DUCREUX, Olivier, F-78430 Louveciennes (FR)
(74) Mandataire: IFP Energies nouvelles
(86) Numéro de dépôt international: PCT/EP2015/063994
(87) Numéro de publication internationale: WO 2016/015923

(56) Documents cités:
- EP-A1- 0 922 482
- FR-A1- 2 486 822
- FR-A1- 2 690 855
- FR-A1- 2 992 233
- US-A- 4 835 338
- Patrick Euzen ET AL: "Alumina" In: "Handbook of Porous solids", 25 avril 2008 (2008-04-25), Wiley-VCH Verlag GmbH, XP055057945, ISBN: 978-3-52-761828-6 pages 1591-1677, DOI: 10.1002/9783527618286, le document en entier

## Description

La présente invention concerne un adsorbant à base d'alumine pour l'élimination de molécules acide telles que le COS et/ou le CO₂ contenu(s) dans des flux d'hydrocarbures liquides ou gazeux. L'invention se rapporte également à des procédés de préparation de cet adsorbant ainsi qu'à son utilisation dans un procédé d'élimination de molécules acides d'un flux hydrocarbures liquides ou gazeux.

### Etat de la technique

Les molécules acides telles que le sulfure de carbonyle (COS), le dioxyde de carbone (CO₂), l'hydrogène sulfuré (H₂S) ou encore le sulfure de carbone (CS₂) sont des contaminants présents dans le gaz naturel, les différents gaz de synthèse et de combustion et les coupes hydrocarbures liquides issues du pétrole ou de la transformation de la biomasse.

Or, ces molécules acides sont néfastes à plusieurs points de vue. D'un point de vue environnemental, il est bien connu que le CO₂ par exemple est l'un des gaz responsable de l'effet de serre. Le COS à son tour, dès lors qu'il est relargué dans l'atmosphère via les effluents gazeux des procédés de raffinage et de pétrochimie, peut être transporté dans la stratosphère où il va altérer la concentration d'ozone via son effet promoteur des réactions photochimiques et entraîner la formation de SO₂. Il va donc simultanément contribuer au réchauffement climatique et amplifier les pluies acides. D'un point de vue industriel, les molécules acides accentuent les phénomènes de corrosion des réacteurs de raffinage et pétrochimie. Par ailleurs, ils contribuent à la présence de soufre dans les produits gaziers et pétroliers et donc peuvent conduire à les rendre hors spécification. Enfin, il s'agit de poisons de nombreux catalyseurs et notamment de ceux de polymérisation d'oléfines.

On cherche ainsi à éliminer les molécules acides, notamment le COS et le CO₂, contenues dans les différents flux d'hydrocarbures.

Le COS présent dans les hydrocarbures liquides et notamment dans les oléfines peut être éliminé de différentes manières.

La première méthode est de traiter l'effluent dans lequel se trouve le COS par des solvants physiques (par exemple la soude) ou chimiques (tels que les amines), c'est-à-dire de transposer les méthodes d'absorption du CO₂ au COS, les deux molécules possédant des propriétés proches dont un caractère acide. L'utilisation de la soude permet comme attendu d'absorber le COS mais avec des vitesses 1000 fois plus lentes que dans le cas du CO₂ (Scott Elliott, Eric Lu, Sherwood Rowland, Rates of mecanisms for the hydrolysis of COS in natural waters, Environ. Sci. Technol., 1989, 23 (4), 458-461). Le même problème de réaction beaucoup plus lente avec le COS qu'avec le CO₂ est rencontré si on veut faire réagir le COS avec des amines primaires ou secondaires (Sharma M.M. Kinetics of Reactions of Carbonyl Sulphide and Carbon Dioxide with Amines and Catalysis by Brönsted Bases of the Hydrolysis of COS., Trans. Faraday Soc., 1965, 61, 681). Par ailleurs, le COS a tendance à réagir de manière irréversible avec les amines entraînant leur dégradation (Bacon T.R., Pearce R.L., Foster W.R., H2S selectivity with carbonyl sulfide removal to less than ppm levels, 1986, Presented at the National Petroleum Refiners Association, 23-25 Mars, Los Angeles). Enfin, avec les amines tertiaires, la réactivité du COS est très faible (Littel R.J., Versteeg G.F., Swaaij W.P.M. Kinetic study of COS with tertiary alkanolamine solutions Experiments in an intensivley Stirred Batch reactor., Ind. Eng. Chem. Res., 1992, 31, 1262-1269.). En somme, cette voie d'élimination du COS n'est pas très efficace et coûterait chère à mettre en oeuvre.

La deuxième méthode souvent évoquée dans la littérature est l'hydrolyse du COS selon la réaction : COS + H₂O → CO₂ + H₂S. Cette réaction est thermodynamiquement favorable et ce d'autant plus qu'on se situe à basse température et que la concentration en COS est importante. Cependant, elle est cinétiquement lente et les taux de conversion peuvent ne pas atteindre 100% notamment quand la température est faible. D'après le brevet US 4,491,516, ce problème peut être résolu par l'ajout d'eau sur l'alumine. Outre le fait qu'à basse température l'eau devient un compétiteur du COS, le principal inconvénient de cette méthode reste qu'elle ne fait que transformer le COS en un autre composé soufré qui devra lui-même être éliminé de l'effluent.

Enfin, la troisième méthode est l'adsorption du COS sur un solide basique. Il s'agit le plus souvent de supports poreux imprégnés d'une ou plusieurs phases dites actives, les supports peuvent néanmoins être utilisés seuls. En tant que support, tout solide poreux classique a été cité : les oxydes dont principalement l'alumine, les charbons actifs, les silice-alumines, les argiles, les zéolithes (Y, X, A, BEA) telles quelles ou échangées par des cations tels que Ag, Cu, Zn, Fe, Co, Ni, les résines basiques. En tant que phase active, on retrouve presque tous les éléments : les alcalins, les alcalino-terreux (sous forme d'oxydes ou de sulfates), les métaux de transition (essentiellement ceux de la première série mais le Pt sous forme sulfure, Co, Mo sont également cités) et les terres rares. Cependant, tous ne présentent pas les qualités requises pour la purification d'hydrocarbures liquides contenant du COS, qui sont une forte capacité d'adsorption et une bonne régénérabilité. Pour les résines échangeuses d'anions basiques (BE 685521 et US 3,282,831), le problème est une teneur résiduelle en COS de l'ordre du ppm ce qui reste trop élevé (EP 0 169 828). Dans le cas des zéolithes et de la purification d'oléfines, le principal problème est la dégradation de l'adsorbant via la formation de coke (S. Watson, R. Kummitt, R.B. Rhinesmith, Oil & Gas Journal, 2003, 101, pp 66-73).

Les solides les plus adaptés sont les oxydes et plus particulièrement l'alumine surtout pour des adsorptions à basse température. Le document US 4,835,338 propose une alumine activée imprégnée par un ou plusieurs métaux alcalins et/ou alcalino-terreux à hauteur de 0,01 à 10% poids en métal pour éliminer le COS d'une charge de propylène liquide. Une fois saturé, l'adsorbant est régénéré par passage d'un gaz chaud (100-300°C). Cependant, les performances du solide se dégradent au fur et à mesure des régénérations (perte de 70% de la capacité initiale au 4^{ème} cycle pour le solide le plus performant donné dans les exemples). Pour remédier à ce problème tout en gardant une température de régénération classique, soit entre 100 et 350°C, le document US 6,843,907 suggère une régénération en deux temps : passage d'un gaz chaud (hydrocarbures, azote ou gaz inerte) contenant un agent hydrolysant tel que de l'eau entre 20 et 6000 ppm puis passage d'un gaz chaud sans ce même agent. La stabilité des performances initiales des alumines testées est maintenue sur 12 cycles. Néanmoins, la mise en oeuvre d'une telle procédure de régénération complique la mise en oeuvre du procédé. Il serait donc intéressant de trouver un adsorbant avec d'aussi bonnes capacités d'adsorption qui sont en plus stables au cours des cycles d'adsorption/désorption sans avoir à ajouter d'agent dans le gaz de régénération.

Le CO₂ quant à lui est un poison des catalyseurs de polymérisation et il est donc à éliminer des coupes oléfiniques et plus particulièrement des coupes éthylène ou propylène quelles que soient leur origine. Elles peuvent notamment être issues du craquage thermique ou catalytique mais également de la conversion d'alcools en oléfines. L'élimination du CO₂ peut se faire par i) absorption par des solvants physiques ou chimiques, ii) utilisation de membranes ou iii) adsorption sur des adsorbants. Les deux premières solutions ne sont pas adaptées aux charges envisagées qui contiennent très peu de CO₂ (moins de 1000 ppmv) et/ou sont chères à mettre en oeuvre pour atteindre les spécifications visées (moins de 1 ppmv). La solution retenue est donc souvent celle de l'utilisation d'un adsorbant solide.

Certains brevets revendiquent l'utilisation de zéolithes telles que la zéolithe A (GB 898,321), la 13X (EP 0 173 501) ou encore la clinoptilolite (US 4,935,580). Bien que ces zéolithes aient toutes une sélectivité d'adsorption en faveur du CO₂, leur capacité en CO₂ à une teneur inférieure à 1000 ppmv dans une coupe oléfinique est très faible. Par ailleurs, elles présentent l'inconvénient d'avoir besoin d'un pré-chargement pour éviter de s'échauffer et de se dégrader. Enfin, elles désactivent souvent rapidement au cours des cycles suite à la formation puis au dépôt de coke à leur surface.

D'autres brevets proposent plutôt l'emploi de carbonates d'alcalins seuls (US 1,831,731) ou en mélange avec de l'alumine (US 3,511,595, US 3,865,924, US 2007/0037702). Cependant, leur réactivité optimale nécessite la présence d'eau pour que la réaction suivante puisse avoir lieu : MCO₃ + H₂O + CO₂ → 2MHCO₃ avec M un cation alcalin. Des alumines promues aux alcalins et/ou alcalino-terreux sont également revendiquées (US 4,433,981, US 6,125,655). Le brevet US 6,125,655 divulgue une alumine calcinée contenant au plus 10 % en poids d'au moins un oxyde métallique alcalin ou alcalino-terreux employée dans un procédé de purification d'un flux d'air contentant du CO₂.

Le brevet FR 2,690,855 divulgue un procédé de préparation d'une composition à base d'alumine avec métaux alcalins comme promoteurs pour l'élimination du HCI d'un fluide. Le brevet FR 2,486,822 divulgue un catalyseur d'alumine activée et d'oxyde de sodium convenant pour enlever le soufre et les composés soufrés des gaz, le catalyseur ayant une quantité d'oxyde de sodium supérieure à 0,50 % en poids (sur la base du produit calciné à 1100°C). En effet, le document US 4,433,981 propose une alumine imprégnée par un ou plusieurs métaux alcalins et/ou métaux alcalino-terreux pour éliminer le CO₂ d'une charge de propylène liquide. Il décrit que les impuretés contenues dans l'alumine n'ont pas d'effet sur les performances de l'adsorbant. Ce document décrit notamment une alumine ayant une teneur en sodium de 4700 ppm poids (équivalent à une teneur de 6300 ppm poids exprimée en Na₂O) imprégnée par un ou plusieurs métaux alcalins et/ou métaux alcalino-terreux. Les adsorbants décrits dans ce document sont préparés par une calcination à relativement haute température menant à la formation d'aluminates de métaux alcalins ou d'aluminates de métaux alcalino-terreux.

### Résumé de l'invention

La présente invention concerne un adsorbant à base d'alumine dopée par un élément alcalin pour l'élimination de molécules acides telles que le COS et/ou le CO₂ contenu(s) dans des flux d'hydrocarbures liquides ou gazeux selon la revendication 1.

La présente invention porte sur un adsorbant comprenant un support d'alumine et au moins un élément alcalin, ledit adsorbant étant obtenu par introduction d'au moins un élément alcalin, identique ou différent au sodium, sur un support d'alumine dont la teneur en sodium, exprimée en équivalent Na₂O, avant introduction du ou des éléments alcalins est comprise entre 1500 et 3500 ppm poids par rapport au poids total du support.

De façon surprenante, la demanderesse a découvert que la présence d'une certaine faible teneur en sodium dans une alumine avant d'être dopée par la suite par au moins un élément alcalin permettait d'obtenir un adsorbant ayant de fortes capacités d'adsorption de molécules acides, et notamment du COS et/ou du CO₂, et qui présente surtout des performances stables au cours des cycles d'adsorption/régénération sans mise en oeuvre de conditions particulières pour la régénération. Sans être lié à aucune théorie, la présence d'une certaine quantité de sodium dans l'alumine avant introduction du ou des éléments alcalins permettrait de combler des défauts de surface de l'alumine et d'éviter la formation de soufre élémentaire dans le cas de l'adsorption de COS, observée sur les solides préparés sur des supports d'alumine non stabilisés par du sodium et qui induit une désactivation dans le temps de l'adsorbant. Mais trop de sodium à la surface du support pourrait réduire l'interaction entre ce support et la phase à introduire (élément alcalin) : le précurseur plutôt basique sera moins attiré par la surface si celle-ci est préalablement rendue trop basique. Ceci pourrait entraîner une moins bonne dispersion de la phase active et donc nuire à l'efficacité de l'adsorbant dans son utilisation ultérieure. L'adsorbant final possédant une phase active à base d'alcalin moins fortement liée à la surface de son support pourrait également être susceptible de relarguer une partie de sa phase active, notamment lors de son utilisation en phase liquide. La présence d'une quantité optimale de sodium dans l'alumine, combinée avec l'introduction du ou des éléments alcalins crée ainsi un effet synergique qui se traduit par une forte capacité d'adsorption et des performances plus stables dans le temps au cours des cycles d'adsorption/régénération.

De plus, la régénération de l'adsorbant selon l'invention peut être effectuée sans avoir recours à l'ajout d'un agent d'hydrolyse dans le gaz de régénération.

Selon une variante, l'élément alcalin est choisi parmi le sodium et le potassium. Selon une variante, la teneur en élément alcalin par rapport au poids total de l'adsorbant est comprise entre 1 et 60% poids dudit élément.

Selon une variante, le support d'alumine avant l'introduction du ou des éléments alcalins a un volume poreux total compris entre 0,3 et 1 cm³.g⁻¹ et une surface spécifique comprise entre 50 et 450 m².g⁻¹.

Selon une variante, l'adsorbant selon l'invention est constitué de potassium et d'un support d'alumine ayant une teneur en sodium, exprimée en équivalent Na₂O, avant introduction du potassium comprise entre 1500 et 3500 ppm poids par rapport au poids total du support.

L'invention concerne également un procédé de préparation de l'adsorbant selon l'invention, comprenant les étapes suivantes :
a) on prépare une solution aqueuse contenant au moins un précurseur d'alcalin,
b) on imprègne un support d'alumine ayant une teneur en sodium, exprimée en équivalent Na₂O, comprise entre 1500 et 3500 ppm poids par rapport au poids total du support par la solution aqueuse obtenue à l'issue de l'étape a),
c) on laisse maturer le support imprégné issu de l'étape b) dans une enceinte close saturée en eau,
d) on sèche le solide issu de l'étape c).

Selon une autre variante, le procédé de préparation de l'adsorbant comprend les étapes suivantes :
a') on mélange un support d'alumine ayant une teneur en sodium, exprimée en équivalent Na₂O, comprise entre 1500 et 3500 ppm poids par rapport au poids total du support, avec une poudre d'au moins un précurseur d'alcalin
b') on broie éventuellement le mélange obtenu à l'issu de l'étape a') à une granulométrie comprise entre 2 et 100 µm,
c') on met en forme le mélange issu de l'étape b') en présence d'eau de manière à obtenir un matériau,
d') on sèche le matériau mis en forme issu de l'étape c').

Selon une autre variante, le procédé de préparation de l'adsorbant comprend les étapes suivantes :
a") on malaxe un support d'alumine ayant une teneur en sodium, exprimée en équivalent Na₂O, comprise entre 1500 et 3500 ppm poids par rapport au poids total du support avec une solution comprenant au moins un précurseur d'alcalin pour obtenir une pâte,
b") on met en forme la pâte obtenue à l'étape a"),
c") on sèche la pâte mise en forme issue de l'étape b").

Selon une variante, on effectue une étape de calcination sous air en atmosphère sèche ou humide à l'issue de l'étape de séchage des trois procédés de préparation.

L'invention concerne également un procédé d'élimination d'une molécule acide d'un flux d'hydrocarbures contenant au moins une molécule acide, dans lequel le flux d'hydrocarbures est mis en contact durant une étape d'adsorption avec un adsorbant selon l'invention.

Selon une variante préférée, la molécule acide à éliminer est le COS et/ou le CO₂. L'étape d'adsorption est mise en oeuvre à une température entre -50 et 100°C, à une pression absolue comprise entre 0,01 MPa et 5 MPa, et à une vitesse volumique horaire comprise entre 50 et 50000 h⁻¹.

Selon une variante, on effectue une étape de régénération de l'adsorbant une fois que l'adsorbant est au moins partiellement saturé en molécules acides.

Selon une variante, l'étape de régénération est effectuée par la mise en contact de l'adsorbant au moins partiellement saturé en molécules acides avec un gaz ou un liquide à une température comprise entre 20 et 500°C, à une pression absolue comprise entre 0,01 MPa et 5 MPa et à une vitesse volumique horaire comprise entre 50 et 50000 h⁻¹.

### Description détaillée de l'invention

La présente invention concerne un adsorbant comprenant un support d'alumine et au moins un élément alcalin, ledit adsorbant étant obtenu par introduction d'au moins un élément alcalin, identique ou différent au sodium, sur un support d'alumine dont la teneur en sodium, exprimée en équivalent Na₂O, avant introduction du ou des éléments alcalins est comprise entre 1500 et 3500 ppm poids par rapport au poids total du support.

L'adsorbant selon l'invention comprend au moins un élément alcalin, identique ou différent au sodium, introduit sur l'alumine contenant elle-même une certaine teneur en sodium. L'élément alcalin introduit est choisi parmi les éléments du groupe IA pour leur capacité à rendre le support d'alumine plus basique. De manière préférée, l'élément alcalin est choisi parmi le sodium (Na) et le potassium (K). De manière particulièrement préférée, l'élément alcalin est le potassium. La demanderesse a en effet remarqué que les performances de l'adsorbant sont d'autant plus améliorées, lorsqu'on utilise le potassium en tant qu'élément alcalin. De manière préférée, la teneur en élément alcalin par rapport au poids total de l'adsorbant est comprise entre 1 et 60% poids dudit élément, de préférence comprise entre 2 et 40% poids, et de manière très préférée comprise entre 2 et 20% poids, voire entre 2 et 15% poids ou entre 4 et 15% poids. La teneur en élément alcalin est mesurée par spectrométrie d'absorption atomique, méthode décrite dans «Analyse Physico-chimique des catalyseurs industriels - Manuel pratique de caractérisation » écrit sous la coordination de John Lynch, Editions Technip 2001, pages 31-46.

L'adsorbant selon l'invention comprend également un support d'alumine ayant une teneur en sodium, exprimée en équivalent Na₂O, comprise entre 1000 et 5000 ppm poids avant introduction du ou des éléments alcalins par rapport au poids total du support. Les caractéristiques du support d'alumine contenant le sodium, mentionnées dans la présente description correspondent aux caractéristiques du support avant que le ou les éléments alcalins aient été introduits sur le support.

La teneur en sodium, exprimée en équivalent Na₂O, dudit support d'alumine avant introduction du ou des éléments alcalins est comprise entre 1500 et 3500 ppm poids par rapport au poids total du support. La teneur en élément alcalin est mesurée par spectrométrie d'absorption atomique, méthode décrite dans l'ouvrage cité ci-dessus.

Sans vouloir se lier à aucune théorie, la présence d'une certaine teneur de sodium dans l'alumine avant introduction du ou des éléments alcalins permettrait de combler des défauts de surface de l'alumine et d'éviter la formation de soufre élémentaire dans le cas de l'adsorption de molécules acides contenant du soufre qui est source de désactivation des sites d'adsorption des gaz acides à la surface de l'adsorbant.

Une teneur insuffisante de sodium dans l'alumine avant introduction du ou des éléments alcalins, c'est-à-dire une teneur inférieure à 1500 ppm par rapport au poids total du support ne permet pas d'observer l'augmentation en capacité d'adsorption et des performances plus stables dans le temps au cours des cycles d'adsorption/régénération. Une teneur insuffisante ne semble donc pas permettre de combler des défauts de surface de l'alumine.

De même, une teneur trop importante de sodium dans l'alumine avant introduction du ou des éléments alcalins, c'est-à-dire une teneur, exprimée en équivalent Na₂O, supérieure à 3500 ppm poids par rapport au poids total du support ne permet pas d'observer l'augmentation en capacité d'adsorption et des performances plus stables dans le temps au cours des cycles d'adsorption/régénération.

Le support d'alumine présente avantageusement un volume poreux total compris entre 0,3 et 1 cm³.g⁻¹, de préférence entre 0,4 et 0,7 cm³.g⁻¹. Le volume poreux total est mesuré par porosimétrie au mercure selon la norme ASTM D4284 avec un angle de mouillage de 140°, telle que décrite dans l'ouvrage Rouquerol F.; Rouquerol J.; Singh K. « Adsorption by Powders & Porous Solids: Principle, methodology and applications », Academic Press, 1999, par exemple au moyen d'un appareil modèle Autopore III™ de la marque Microméritics™.

La surface spécifique du support d'alumine est avantageusement comprise entre 50 et 450 m².g⁻¹, de préférence entre 100 et 400 m².g⁻¹, de manière plus préférée entre 150 et 370 m².g⁻¹, de manière encore plus préférée entre 200 et 350 m².g⁻¹. La surface spécifique est déterminée dans la présente invention par la méthode B.E.T selon la norme ASTM D3663, méthode décrite dans le même ouvrage cité ci-dessus.

De manière préférée, ledit support d'alumine présente une structure cristallographique du type alumine de transition telle qu'une alumine chi, gamma, delta, thêta, rho ou êta, seule ou en mélange. De manière plus préférée, l'alumine est une alumine de transition chi, gamma ou delta, seule ou en mélange. Les phases cristallographiques sont généralement obtenues à partir de diffractogrammes obtenus par diffraction des rayons X.

De manière préférée, l'adsorbant selon l'invention est constitué de potassium et d'un support d'alumine ayant une teneur en sodium, exprimée en équivalent Na₂O, avant introduction du ou des éléments alcalins comprise entre 1500 et 3500 ppm poids par rapport au poids total du support.

Le support d'alumine ayant une teneur en sodium, exprimée en équivalent Na₂O, avant introduction du ou des éléments alcalins comprise entre 1500 et 3500 ppm poids par rapport au poids total du support peut être synthétisé par différentes méthodes connues par l'homme du métier, par exemple par les méthodes décrites ci-après.

Selon la première méthode de synthèse du support, on effectue une déshydratation rapide d'un précurseur de type trihydroxyde d'aluminium (Al(OH)₃) (autrement dénommé hydrargillite ou gibbsite) par exemple issu du procédé couramment nommé "Bayer". Puis on effectue une mise en forme par exemple par granulation, puis un traitement hydrothermal et enfin une calcination qui conduit à l'obtention d'alumine. Cette méthode est notamment détaillée dans le document P. Euzen, P. Raybaud, X. Krokidis, H. Toulhoat, J.L. Le Loarer, J.P. Jolivet, C. Froidefond, Alumina, in Handbook of Porous Solids, Eds F. Schüth, K.S.W. Sing, J. Weitkamp, Wiley-VCH, Weinheim, Germany, 2002, pp. 1591-1677. Cette méthode permet de produire une alumine couramment nommée "alumine flash".

Selon la deuxième méthode de synthèse du support, on met en oeuvre un procédé d'obtention de gel constitué d'un précurseur de type gamma-oxy(hydroxyde) d'aluminium (AlO(OH) - autrement dénommé boehmite - présentant des surfaces spécifiques élevées comprises entre 150 et 600 m²/g. Ensuite, on met en forme le gel, par exemple par malaxage-extrusion. Puis on effectue une série de traitements thermiques ou hydrothermaux au produit conduisant à l'obtention de l'alumine. Le gel de boehmite peut par exemple être obtenu par précipitation des solutions basiques et/ou acides de sels d'aluminium induite par changement de pH ou toute autre méthode connue de l'homme de métier. Cette méthode est notamment décrite dans le même ouvrage cité ci-dessus. Cette méthode permet de produire une alumine couramment nommée "alumine gel".

Le sodium présent dans le support d'alumine est généralement introduit pendant ou après la synthèse de l'alumine, mais toujours avant l'introduction du ou des éléments alcalins. Plus particulièrement, le sodium présent dans le support peut être déjà présent dans les précurseurs aluminiques des deux méthodes de préparation d'alumine décrites ci-dessus, c'est-à-dire dans le précurseur de type trihydroxyde d'aluminium (Al(OH)₃) ou dans le précurseur de type gamma-oxy(hydroxyde) d'aluminium (AlO(OH)). Le sodium présent dans le support d'alumine peut également être introduit en quantité souhaitée dans le support soit lors de la mise en forme du support, par exemple lors de l'étape de granulation dans la synthèse d'une alumine flash ou dans l'étape de malaxage-extrusion dans la synthèse d'une alumine gel, soit encore par imprégnation du précurseur aluminique.

De manière préférée, on met en oeuvre la première méthode de synthèse du support d'alumine de l'adsorbant selon l'invention. Il est en effet connu qu'une alumine préparée par déshydratation rapide d'un précurseur de type trihydroxyde (alumine flash) contient en règle générale une teneur en sodium plus importante qu'une alumine préparée à partir d'un gel d'alumine.

Le support d'alumine, et donc l'adsorbant selon l'invention, peut se présenter sous forme d'une pluralité d'éléments, chaque élément ayant la forme de bille, de cylindre, d'extrudé multilobé (par exemple trilobe ou quadrilobe), de roue de charrette, de cylindre creux ou toute autre forme géométrique utilisée par l'homme de métier. Chacun des éléments constituant l'adsorbant répond aux caractéristiques de l'adsorbant selon l'invention. De manière plus préférée, le support d'alumine, et donc l'adsorbant selon l'invention, se présente sous forme d'une pluralité de billes de diamètres compris entre 0,4 et 100 mm, de préférence compris entre 0,5 et 50 mm, de manière plus préférée compris entre 0,5 et 10 mm.

### Procédé de préparation de l'adsorbant

L'invention concerne également des procédés de préparation de l'adsorbant selon l'invention. L'adsorbant selon l'invention peut être préparé en déposant l'élément alcalin sur le support poreux décrit ci-dessus, par des voies de synthèses connues de l'homme de métier, par exemple par imprégnation à sec ou humide, par co-granulation, ou par co-malaxage.

Selon une première variante, le procédé de préparation de l'adsorbant selon l'invention comprend les étapes suivantes :
a) on prépare une solution aqueuse contenant au moins un précurseur d'alcalin,
b) on imprègne un support d'alumine ayant une teneur en sodium, exprimée en équivalent Na₂O, comprise entre 1500 et 3500 ppm poids par rapport au poids total du support par la solution aqueuse obtenue à l'issue de l'étape a),
c) on laisse maturer le support imprégné issu de l'étape b) dans une enceinte close saturée en eau,
d) on sèche le solide issu de l'étape c).

Selon une variante du procédé, le séchage peut être suivi d'une étape e) de calcination sous air sec ou humide.

Selon l'étape a) de la première variante de procédé de préparation, on prépare une solution aqueuse contenant au moins un précurseur d'alcalin.

Le précurseur du ou des éléments alcalins peut être n'importe quel sel d'alcalin soluble dans l'eau ; de manière préférée le précurseur de l'élément alcalin est choisi parmi l'hydroxyde, le nitrate et le carbonate de l'élément alcalin. De manière très préférée, le précurseur du ou des élément(s) alcalins est l'hydroxyde correspondant. Lorsque l'élément alcalin est le sodium, le précurseur est de préférence la soude (NaOH) ; lorsque l'élément alcalin est le potassium, le précurseur est de préférence la potasse (KOH).

Les quantités du ou des éléments alcalins introduites dans la solution sont choisies de telle manière que la teneur totale en élément alcalin par rapport au poids total de l'adsorbant est comprise entre 1 et 60% poids dudit élément, de préférence comprise entre 2 et 40% poids, et de manière très préférée comprise entre 2 et 20% poids, voire entre 2 et 15% poids ou entre 5 et 15% poids.

Selon l'étape b) de la première variante de procédé de préparation, on imprègne un support d'alumine ayant une teneur en sodium, exprimée en équivalent Na₂O, comprise entre 1000 et 5000 ppm poids par rapport au poids total du support par la solution aqueuse obtenue à l'issue de l'étape a).

L'imprégnation du ou des éléments alcalins peut être réalisée selon toutes les méthodes connues de l'Homme du métier, en particulier par imprégnation à sec, c'est-à-dire par imprégnation où le volume de la solution d'imprégnation correspond exactement au volume de reprise en eau du support, c'est à dire au volume poreux accessible du solide. De façon préférée, le ou les éléments alcalins sont déposés par imprégnation à sec de leurs précurseurs associés sur le support d'alumine ayant une teneur en sodium, exprimée en équivalent Na₂O, avant imprégnation comprise entre 1500 et 3500 ppm poids par rapport au poids total du support.

L'imprégnation peut se faire via une seule étape d'imprégnation. L'imprégnation peut aussi être avantageusement réalisée via au moins deux cycles d'imprégnation. Dans ce cas, chaque imprégnation est avantageusement suivie d'une maturation, d'un séchage et éventuellement d'une calcination dans les conditions opératoires décrites ci-dessous pour les étapes c), d) et e).

Selon l'étape c) de la première variante de procédé de préparation, on laisse maturer le support imprégné issu de l'étape b) dans une enceinte close saturée en eau.

L'étape c) de maturation du support imprégné est menée dans une enceinte close saturée en eau, de préférence à une température comprise entre 20°C et 60°C, et pendant une durée comprise de préférence entre 0,5 heure et 8 heures. L'étape c) de maturation se fait généralement à température ambiante.

Selon l'étape d) de la première variante de procédé de préparation, on sèche le solide issu de l'étape c).

L'étape d) de séchage peut être réalisée à l'air, à une température pouvant être comprise entre 70°C et 250°C, de préférence entre 80°C et 200°C, généralement pendant une durée comprise de préférence entre 1 et 24 heures.

Dans une variante de la première variante de procédé selon l'invention, la préparation de l'adsorbant comporte à l'issu de l'étape d) une étape supplémentaire e) comprenant une calcination sous air en atmosphère sèche ou humide. La calcination est généralement effectuée sous air à une température typiquement comprise entre 280°C et 550°C en atmosphère sèche ou humide, de préférence à une température comprise entre 300°C et 500°C, et de manière particulièrement préférée à une température comprise entre 350 et 450°C. La calcination est effectuée à une température telle qu'on n'observe pas la formation d'aluminates de métaux alcalins (c'est-à-dire à une température relativement basse). De manière préférée lors de l'étape e), le solide issu de l'étape d) est calciné sous air contenant une humidité relative à 25°C comprise entre 10% et 80%, de manière préférée entre 15% et 50%.

Selon une deuxième variante, le procédé de préparation de l'adsorbant selon l'invention peut être effectué par co-granulation. La co-granulation consiste à mettre en forme un mélange de poudres. Selon cette variante, le procédé de préparation de l'adsorbant selon l'invention comprend les étapes suivantes :
a') on mélange un support d'alumine ayant une teneur en sodium, exprimée en équivalent Na₂O, comprise entre 1500 et 3500 ppm poids par rapport au poids total du support, avec une poudre d'au moins un précurseur d'alcalin,
b') on broie éventuellement le mélange obtenu à l'issu de l'étape a') à une granulométrie comprise entre 2 et 100 µm,
c') on met en forme le mélange issu de l'étape b') en présence d'eau de manière à obtenir un matériau,
d') on sèche le matériau mis en forme issu de l'étape c').

Le précurseur alcalin utilisé en tant que poudre dans l'étape a') peut être un des précurseurs alcalins décrits pour l'étape a) du procédé de préparation via l'imprégnation. Il est introduit dans les quantités indiquées à l'étape a).

L'étape c') de mise en forme est réalisée selon toute technique connue de l'Homme du métier, par exemple les méthodes de mise en forme par extrusion, par pastillage, par la méthode de la goutte d'huile (égouttage) ou par granulation au plateau tournant. De préférence, la mise en forme est réalisée par granulation au plateau tournant.

L'étape d') de séchage est effectuée dans les conditions opératoires décrites pour l'étape d) du procédé de préparation par imprégnation.

Selon une variante de la deuxième variante de procédé de préparation (co-granulation), le séchage peut être suivi d'une étape e') de calcination sous air sec ou humide dans les conditions telles que décrites pour l'étape e) du procédé de préparation par imprégnation.

Selon une troisième variante, le procédé de préparation de l'adsorbant selon l'invention peut être effectué par co-malaxage. Selon cette variante, le procédé de préparation de l'adsorbant selon l'invention comprend les étapes suivantes :
a") on malaxe un support d'alumine ayant une teneur en sodium, exprimée en équivalent Na₂O, comprise entre 1500 et 3500 ppm poids par rapport au poids total du support avec une solution comprenant au moins un précurseur d'alcalin pour obtenir une pâte,
b") on met en forme la pâte obtenue à l'étape a"),
c") on sèche la pâte mise en forme issue de l'étape b").

Le précurseur alcalin utilisé dans la solution de l'étape a") peut être un des précurseurs alcalins décrits pour l'étape a) du procédé de préparation via l'imprégnation. Il est introduit dans les quantités indiquées à l'étape a).

L'étape b") de mise en forme est réalisée selon toute technique connue de l'Homme du métier, par exemple les méthodes de mise en forme par extrusion, par pastillage, par la méthode de la goutte d'huile (égouttage) ou par granulation au plateau tournant. De préférence, la mise en forme est réalisée par extrusion. L'étape c") de séchage est effectuée dans les conditions opératoires décrites pour l'étape d) du procédé de préparation par imprégnation.

Selon une variante de la troisième variante de procédé de préparation (co-malaxage), le séchage peut être suivi d'une étape d") de calcination sous air sec ou humide dans les conditions telles que décrites pour l'étape e) du procédé de préparation par imprégnation.

### Procédé d'élimination de molécules acides

L'invention concerne également un procédé d'élimination d'une molécule acide d'un flux d'hydrocarbures contenant au moins une molécule acide, dans lequel le flux d'hydrocarbures est mis en contact durant une étape d'adsorption avec un adsorbant selon l'invention.

L'adsorbant selon l'invention est particulièrement bien adapté pour capter le COS et/ou le CO₂ contenus dans un flux liquide ou gazeux, notamment à basse température.

L'adsorbant, par exemple sous forme de lit fixe disposé dans un réacteur, est mis en contact avec le flux liquide ou gazeux à traiter.

Industriellement, l'élimination des molécules acides d'un flux d'hydrocarbures gazeux ou liquide est réalisée par une circulation du flux à traiter au travers de lits fixes remplis de l'adsorbant. L'impureté à éliminer, ici notamment le COS et/ou le CO₂, est alors retenue au sein ou à la surface de l'adsorbant et le flux évacué est alors purifié.

De manière préférée, la molécule acide à éliminer est le COS et/ou le CO₂.

Le flux d'hydrocarbures contenant du COS peut être un flux liquide ou gazeux, de préférence un flux liquide. Le flux d'hydrocarbures traité dans le procédé selon l'invention peut être un flux contenant des hydrocarbures liquides ou gazeux saturés ou insaturés possédant de 1 à 6 atomes de carbone. Le flux liquide ou gazeux peut être par exemple une coupe oléfinique issue du craquage catalytique, par exemple une coupe de propylène. De manière préférée, le flux d'hydrocarbures est un flux de propylène liquide.

Le flux d'hydrocarbures à traiter selon l'invention contient du COS en proportions variables. Par exemple le flux d'hydrocarbures à traiter, notamment un flux de propylène liquide, contient entre 10 et 200 ppm poids de COS.

Le flux d'hydrocarbures contenant du CO₂ peut être un flux liquide ou gazeux, de préférence un flux gazeux. Le flux d'hydrocarbures traité dans le procédé selon l'invention peut être un flux contenant des hydrocarbures liquides ou gazeux saturés ou insaturés possédant de 1 à 6 atomes de carbone. Le flux liquide ou gazeux peut être par exemple une coupe oléfinique issue du craquage catalytique, par exemple une coupe d'éthylène. Il peut également être issu de procédé de déshydratation d'alcool, notamment de déshydratation d'éthanol ou de butanol. De manière préférée, le flux d'hydrocarbures est un flux de d'éthylène gazeux ou de butène liquide.

La teneur en CO₂ est généralement comprise entre 10 et 500 ppm molaire de CO₂ dans le flux d'hydrocarbures.

Si présent, l'H₂S sera au moins partiellement adsorbé par l'adsorbant selon l'invention.

La mise en contact du flux d'hydrocarbures avec l'adsorbant selon l'invention est réalisée à une température généralement comprise entre -50 et 100°C, de préférence entre 0 et 70°C, et de manière très préférée entre 20 et 50°C, et à une pression absolue par exemple comprise entre 0,01 MPa et 20 MPa (0,1 et 200 bars), de préférence entre 0,05 MPa et 10 MPa (0,5 et 100 bars) et de manière très préférée entre 0,1 MPa et 5 MPa (1 et 50 bars).

Avantageusement, lors de la mise en contact du flux d'hydrocarbures avec l'adsorbant, la V.V.H. (Vitesse Volumique Horaire, soit le volume de l'effluent par volume d'adsorbant et par heure) mise en oeuvre dans le procédé de d'élimination selon l'invention est comprise entre 50 et 50000 h⁻¹.

Le flux d'hydrocarbures peut éventuellement contenir de l'eau. De préférence, si le flux d'hydrocarbures contient de l'eau, il est préalablement séché selon toute méthode connue de l'homme de l'art. De préférence, le flux d'hydrocarbures est séché par passage sur un lit de zéolithe, par exemple de type LTA échangée au sodium ou au potassium.

Le contact avec l'adsorbant permet avantageusement de capter le COS et/ou le CO₂ dans le flux à traiter, et d'obtenir un flux ayant une teneur en COS et/ou CO₂ réduite par rapport à la teneur du flux initial, voire d'éliminer totalement le COS et/ou CO₂.

Avantageusement, la diminution de teneur totale en COS et/ou CO₂ entre le flux d'hydrocarbures avant traitement et le flux d'hydrocarbures obtenu après traitement avec l'adsorbant selon l'invention peut représenter au moins 90%, préférentiellement au moins 95%, et plus préférentiellement au moins 99%. Selon une variante, on effectue une étape de régénération de l'adsorbant une fois que l'adsorbant est au moins partiellement saturé en molécules acides, notamment en COS et/ou en CO₂.

L'adsorbant au moins partiellement saturé en molécules acides, notamment en COS et/ou en CO₂ est régénéré par passage d'un gaz ou d'un liquide. Le gaz peut être de l'air, de l'azote, des hydrocarbures gazeux secs ou humides. Le liquide peut être un hydrocarbure.

La mise en contact du gaz ou du liquide de régénération avec l'adsorbant selon l'invention peut être réalisée à une température généralement comprise entre 20 et 500°C, de préférence entre 50 et 350°C, et de manière très préférée entre 100 et 300°C, et à une pression absolue par exemple comprise entre 0,01 MPa et 20 MPA (0,1 et 200 bars), de préférence entre 0,05 MPa et 10 MPa (0,5 et 100 bars), et de manière très préférée entre 0,1 MPa et 5 MPa (1 et 50 bars). Avantageusement, lors de la mise en contact du gaz ou du liquide de régénération avec l'adsorbant, la V.V.H. (Vitesse Volumique Horaire, soit le volume de l'effluent par volume d'adsorbant et par heure) mise en oeuvre dans la phase de régénération selon l'invention est comprise entre 50 et 50000 h⁻¹. Cette étape de régénération se fait préférentiellement à contre-courant à la circulation du flux d'hydrocarbures lors de l'étape d'adsorption.

L'étape de régénération peut être mise en oeuvre une fois l'adsorbant complètement saturé ou partiellement saturé. De manière préférée, la phase de régénération est mise en oeuvre dès que des molécules acides sont détectées dans l'effluent de sortie.

L'étape de régénération peut être mise en oeuvre successivement ou parallèlement à l'étape d'adsorption si le procédé prévoit deux lits d'adsorbants en parallèle. De manière préférée, l'adsorbant est disposé en deux lits en parallèle de façon à ce que la régénération d'un adsorbant soit mise en oeuvre pendant l'étape d'adsorption d'un autre adsorbant.

Les exemples présentés ci-après permettent d'illustrer le fonctionnement et les avantages de la présente l'invention.

### Exemple A. Préparation d'un adsorbant A₁ selon l'invention

L'adsorbant A₁ a été préparé par imprégnation à sec d'un support de type alumine flash préparé par granulation par une solution de NaOH. L'imprégnation à sec consiste à mettre en contact le support avec un volume de solution d'imprégnation qui corresponde exactement à son volume poreux disponible.

Le support choisi possède un volume poreux de 0,5 mL/g, une surface spécifique de 341 m²/g et une teneur en sodium, exprimée en équivalent Na₂O, avant imprégnation de 2610 ppm poids. La surface spécifique est déterminée dans la présente invention par la méthode B.E.T selon la norme ASTM D3663, méthode décrite dans le même ouvrage Rouquerol F.; Rouquerol J.; Singh K. « Adsorption by Powders & Porous Solids: Principle, methodology and applications », Academic Press, 1999. Le volume poreux total est mesuré par porosimétrie au mercure selon la norme ASTM D4284 avec un angle de mouillage de 140°, telle que décrite dans l'ouvrage cité ci-dessus, par exemple au moyen d'un appareil modèle Autopore III™ de la marque Microméritics™. La teneur en Na₂O avant et après imprégnation est mesurée par spectroscopie d'absorption atomique selon la méthode décrite dans l'ouvrage de J. Lynch « Analyse Physico-chimique des catalyseurs industriels - Manuel pratique de caractérisation », Editions Technip, 2001.

Pour obtenir un adsorbant A₁ après imprégnation à 6,5% poids en sodium, exprimée en équivalent Na₂O, à partir de 10 g de solution, on procède de la manière suivante :
a) préparation de 50 mL de solution par dissolution de 8,84 g de NaOH dans 50 g d'eau ;
b) imprégnation au goutte à goutte de 5mL de la solution de soude préparée à l'étape a) qui est versée au moyen d'une burette sur le support alumine placé dans un drageoir tournant,
c) maturation du support imprégné dans une enceinte close saturée en eau à 20°C, pendant 3 h,
d) séchage du solide à 90°C pendant 3h
e) calcination du solide sous air à 350°C pendant 1h.

### Exemple B. Préparation d'un adsorbant A₂ selon l'invention

L'adsorbant A₂ a été préparé par imprégnation à sec du même support que celui de l'exemple A par une solution de KOH.

Pour obtenir un adsorbant A₂ après imprégnation à 6,5% poids en potassium, exprimée en équivalent K₂O, à partir de 10 g de solution, on procède de la manière suivante :
a) préparation de 50 mL de solution par dissolution de 11,75 g de KOH dans 50 g d'eau;
b) imprégnation au goutte à goutte de 5 mL de la solution de potasse préparée à l'étape a) qui est versée au moyen d'une burette sur le support alumine placé dans un drageoir tournant,
c) maturation du support imprégné dans une enceinte close saturée en eau à 20°C, pendant 3 h,
d) séchage du solide à 90°C pendant 3h
e) calcination du solide sous air à 350°C pendant 1h.

### Exemple C. Préparation d'un adsorbant A₃ selon l'invention

L'adsorbant A₃ a été préparé par co-granulation du même support que celui de l'exemple A avec du carbonate de potassium.

Pour obtenir un adsorbant A₃ à 6,5% poids en potassium, exprimée en équivalent K₂O, à partir de poudres d'alumine et de carbonate de potassium, on procède de la manière suivante :
a) mélange intime de 9,44 g du support et de 4,68 g de K₂CO₃,
b) broyage du mélange entre 50 et 100 µm,
c) granulation dans un plateau tournant en présence d'eau pulvérisée,
d) séchage du co-granulé à 90°C pendant 3 h,
e) calcination du co-granulé sous air à 350°C pendant 1h.

### Exemple D. Préparation d'un adsorbant A₄ (comparatif)

L'adsorbant A₄ a été préparé par imprégnation à sec d'un support de type alumine flash préparé par granulation par une solution de NaOH.

Le support choisi possède un volume poreux de 0,6 mL/g, une surface spécifique 313 m²/g et une teneur en sodium, exprimée en équivalent Na₂O, avant imprégnation de 760 ppm poids.

Pour obtenir un adsorbant A₄ après imprégnation à 6,5% poids en sodium, exprimée en équivalent Na₂O, à partir de 10 g de solution, on procède de la manière suivante :
a) préparation de 50 mL de solution par dissolution de 7,52 g de NaOH dans 50 g d'eau ;
b) imprégnation au goutte à goutte de 6 mL de la solution de soude préparée à l'étape a) qui est versée au moyen d'une burette sur le support alumine placé dans un drageoir tournant,
c) maturation du support imprégné dans une enceinte close saturée en eau à 20°C, pendant 3 h,
d) séchage du solide à 90°C pendant 3h
e) calcination du solide sous air à 350°C pendant 1h.

### Exemple E. Préparation d'un adsorbant A₅ (comparatif)

L'adsorbant A₅ a été préparé par imprégnation à sec d'un support de type alumine gel préparée par malaxage-extrusion par une solution de NaOH.

Le support choisi possède un volume poreux de 0,7 mL/g, une surface spécifique 282 m²/g et une teneur en sodium, exprimée en équivalent Na₂O, avant imprégnation de 570 ppm poids.

Pour obtenir un adsorbant A₅ après imprégnation à 6,5% poids en sodium, exprimée en équivalent Na₂O, à partir de 10 g de solution, on procède de la manière suivante :
a) préparation de 50 mL de solution par dissolution de 6,44 g de NaOH dans 50 g d'eau;
b) imprégnation au goutte à goutte de 7mL de la solution de soude préparée à l'étape a) qui est versée au moyen d'une burette sur le support alumine placé dans un drageoir tournant,
c) maturation du support imprégné dans une enceinte close saturée en eau à 20°C, pendant 3 h,
d) séchage du solide à 90°C pendant 3h
e) calcination du solide sous air à 350°C pendant 1h.

### Exemple F. Préparation d'un adsorbant A₆ (comparatif)

L'adsorbant A₆ a été préparé par imprégnation à sec d'un support de type alumine flash préparé par granulation par une solution de NaOH.

Le support choisi possède un volume poreux de 0,35 mL/g, une surface spécifique 267 m²/g et une teneur en sodium, exprimée en équivalent Na₂O, avant imprégnation de 6340 ppm poids.

Pour obtenir un adsorbant A₆ après imprégnation à 6,5% poids en sodium, exprimée en équivalent Na₂O, à partir de 10 g de solution, on procède de la manière suivante :
a) préparation de 50 mL de solution par dissolution de 12,79 g de NaOH dans 50 g d'eau ;
b) imprégnation au goutte à goutte de 3,5 mL de la solution de soude préparée à l'étape a) qui est versée au moyen d'une burette sur le support alumine placé dans un drageoir tournant,
c) maturation du support imprégné dans une enceinte close saturée en eau à 20°C, pendant 3 h,
d) séchage du solide à 90°C pendant 3h
e) calcination du solide sous air à 350°C pendant 1h.

### Exemple G. Tests des capacités d'adsorption de COS des différents adsorbants A₁, A₂ A₃ A₄, A₅ et A₆

Les performances d'adsorption de COS des adsorbants ainsi préparés sont testées dans un réacteur en lit fixe de volume 1,5 cm³.

Dans un premier temps, les solides sont activés à 290°C sous 10 NL/h d'azote pendant 12h. Puis, un flux liquide de propylène contenant 50 ppm poids de COS est passé à travers le lit d'adsorbants à un débit de 50 g/h, à une température de 50°C et une pression de 2 MPa. La concentration en COS est mesurée en sortie de réacteur au moyen d'un chromatogramme équipé d'un détecteur spécifique soufre (PFPD). Ces conditions opératoires sont appliquées sur chaque échantillon d'adsorbant jusqu'à ce que celui-ci soit saturé. L'adsorbant est considéré comme saturé lorsque les concentrations en COS en sortie du réacteur deviennent égales à celle de la charge. L'adsorbant est alors chauffé à 290°C sous flux d'azote (5NL/h) pour désorber le COS adsorbé à l'étape précédente pendant environ 10h. Une fois régénéré, l'adsorbant est de nouveau soumis au flux de propylène liquide contenant 50 ppm poids de COS dans les mêmes conditions que celles décrites ci-dessus. Pour chaque adsorbant, quatre cycles d'adsorption/désorption sont réalisés successivement.

Pour chaque cycle, il est possible d'évaluer la quantité de COS qui s'est chimisorbée sur chaque adsorbant en réalisant un bilan matière sur le COS entre l'entrée et la sortie du réacteur sur l'intégralité de la durée du test. Les capacités relatives à saturation en COS (qₛₐₜ en g de COS pour 100 g d'adsorbant) des différents adsorbants sont présentées dans le tableau 1.

**Tableau 1**

| Adsorbant | Teneur en Na (ppm pds Na₂O) du support avant introduction de l'élément alcalin | qₛₐₜ (g /100 g d'adsorbant) | | | |
|---|---|---|---|---|---|
| | | Cycle 1 | Cycle 2 | Cycle 3 | Cycle 4 |
| A₁ | 2610 | 2,05 | 1,72 | 1,70 | 1,71 |
| A₂ | 2610 | 2,58 | 2,15 | 2,18 | 2,17 |
| A₃ | 2610 | 2,27 | 1,78 | 1,86 | 1,81 |
| A₄ | 760 | 2,30 | 1,46 | 1,15 | 0,97 |
| A₅ | 570 | 2,62 | 1,39 | 0,94 | 0,78 |
| A₆ | 6340 | 1,34 | 0,77 | 0,73 | 0,76 |

Ces exemples montrent qu'au deuxième cycle comparativement au premier cycle, les adsorbants préparés perdent tous un peu de capacité d'adsorption en COS ce qui est attribué à une adsorption irréversible d'une partie du COS sur les sites d'adsorption les plus forts. Puis au-delà, les performances en adsorption du COS se stabilisent uniquement pour les adsorbants préparés selon l'invention. Pour les deux adsorbants comparatifs préparés à partir de supports non suffisamment stabilisés par du sodium, les capacités d'adsorption continuent à diminuer au cours des cycles d'adsorption/régénération après le 2^{ème} cycle. Pour l'adsorbant comparatif ayant une teneur en sodium trop élevée (supérieure à 5000 ppm poids), les performances sont stables au-delà du deuxième cycle mais sont très nettement moins bonnes. Ceci est attribué à une moins bonne dispersion de la phase active à la surface du solide entraînant une moins bonne accessibilité des sites d'adsorption.

### Exemple H. Tests des capacités d'adsorption de CO₂ des différents adsorbants A₁, A₂, A₃, A₄, A₅ et A₆

Les performances d'adsorption de CO₂ des adsorbants ainsi préparés sont testées dans un réacteur en lit fixe de volume 1,5 cm³.

Dans un premier temps, les solides sont activés à 290°C sous 10 NL/h d'azote pendant 12h. Puis, un flux gazeux d'éthylène contenant 200 ppm molaire de CO₂ est passé à travers le lit d'adsorbants à un débit de 5 NL/h, à une température de 50°C et une pression de 0,1 MPa. La concentration en CO₂ est mesurée en sortie de réacteur au moyen d'un chromatogramme équipé d'un cathétomètre. Ces conditions opératoires sont appliquées sur chaque échantillon d'adsorbant jusqu'à ce que celui-ci soit saturé. L'adsorbant est considéré comme saturé lorsque les concentrations en CO₂ en sortie du réacteur deviennent égales à celle de la charge. L'adsorbant est alors chauffé à 290°C sous flux d'azote (5NL/h) pour désorber le CO₂ adsorbé à l'étape précédente pendant environ 10h. Une fois régénéré, l'adsorbant est de nouveau soumis au flux de d'éthylène gazeux contenant 200 ppm molaire de CO₂ dans les mêmes conditions que celles décrites ci-dessus. Pour chaque adsorbant, quatre cycles d'adsorption/désorption sont réalisés successivement.

Pour chaque cycle, il est possible d'évaluer la quantité de CO₂ qui s'est chimisorbé sur chaque adsorbant en réalisant un bilan matière sur le CO₂ entre l'entrée et la sortie du réacteur sur l'intégralité de la durée du test. Les capacités relatives à saturation en CO₂ (qₛₐₜ en g de CO₂ pour 100 g d'adsorbant) des différents échantillons de masses de captation sont présentées dans le tableau 2.

**Tableau 2**

| Adsorbant | Teneur en Na (ppm pds Na₂O) du support avant introduction de l'élément alcalin | qₛₐₜ | | | |
|---|---|---|---|---|---|
| | | Cycle 1 | Cycle 2 | Cycle 3 | Cycle 4 |
| A₁ | 2610 | 1,88 | 1,85 | 1,82 | 1,87 |
| A₂ | 2610 | 2,44 | 2,37 | 2,42 | 2,40 |
| A₃ | 2610 | 2,21 | 2,09 | 2,18 | 2,13 |
| A₄ | 760 | 2,26 | 1,97 | 1,86 | 1,74 |
| A₅ | 570 | 2,46 | 2,35 | 1,97 | 1,80 |
| A₆ | 6340 | 1,60 | 1,51 | 1,45 | 1,49 |

Ces exemples montrent que les adsorbants selon l'invention sont ceux qui ont les performances stabilisées les plus hautes. Les adsorbants A₄ et A₅ qui ne possèdent pas assez de sodium avant imprégnation perdent en capacité au cours des cycles. L'adsorbant A₆ ayant une teneur en sodium trop élevée (supérieure à 5000 ppm poids), présente des performances stables mais nettement moins bonnes. Ceci est attribué à une moins bonne dispersion de la phase active à la surface du solide entraînant une moins bonne accessibilité des sites d'adsorption.

## Revendications

1. Adsorbant comprenant un support d'alumine et au moins un élément alcalin, ledit adsorbant étant obtenu par introduction d'au moins un élément alcalin, identique ou différent au sodium, sur un support d'alumine dont la teneur en sodium, exprimée en équivalent Na₂O, avant introduction du ou des éléments alcalins est comprise entre 1500 et 3500 ppm poids par rapport au poids total du support.

2. Adsorbant selon la revendication 2, dans lequel l'élément alcalin est choisi parmi le sodium et le potassium.

3. Adsorbant selon les revendications 1 ou 2, dans lequel la teneur en élément alcalin par rapport au poids total de l'adsorbant est comprise entre 1 et 60% poids dudit élément.

4. Adsorbant selon l'une des revendications 1 à 3, dans lequel le support d'alumine avant l'introduction du ou des éléments alcalins a un volume poreux total compris entre 0,3 et 1 cm³.g⁻¹ et une surface spécifique comprise entre 50 et 450 m².g⁻¹.

5. Adsorbant selon l'une des revendications 1 à 4, lequel est constitué de potassium et du support d'alumine ayant une teneur en sodium, exprimée en équivalent Na₂O, avant introduction du potassium comprise entre 1500 et 3500 ppm poids par rapport au poids total du support.

6. Procédé de préparation de l'adsorbant tel que défini dans l'une des revendications 1 à 5, comprenant les étapes suivantes :
a) on prépare une solution aqueuse contenant au moins un précurseur d'alcalin,
b) on imprègne un support d'alumine ayant une teneur en sodium, exprimée en équivalent Na₂O, comprise entre 1500 et 3500 ppm poids par rapport au poids total du support par la solution aqueuse obtenue à l'issue de l'étape a),
c) on laisse maturer le support imprégné issu de l'étape b) dans une enceinte close saturée en eau,
d) on sèche le solide issu de l'étape c).

7. Procédé de préparation de l'adsorbant tel que défini dans l'une des revendications 1 à 5, comprenant les étapes suivantes :
a') on mélange un support d'alumine ayant une teneur en sodium, exprimée en équivalent Na₂O, comprise entre 1500 et 3500 ppm poids par rapport au poids total du support, avec une poudre d'au moins un précurseur d'alcalin b') on broie éventuellement le mélange obtenu à l'issu de l'étape a') à une granulométrie comprise entre 2 et 100 µm,
c') on met en forme le mélange issu de l'étape b') en présence d'eau de manière à obtenir un matériau,
d') on sèche le matériau mis en forme issu de l'étape c').

8. Procédé de préparation de l'adsorbant tel que défini dans l'une des revendications 1 à 5, comprenant les étapes suivantes :
a") on malaxe un support d'alumine ayant une teneur en sodium, exprimée en équivalent Na₂O, comprise entre 1500 et 3500 ppm poids par rapport au poids total du support avec une solution comprenant au moins un précurseur d'alcalin pour obtenir une pâte,
b") on met en forme la pâte obtenue à l'étape a"),
c") on sèche la pâte mise en forme issue de l'étape b").

9. Procédé selon les revendications 6 à 8, dans lequel on effectue une étape de calcination à une température comprise entre 280 et 550°C sous air en atmosphère sèche ou humide à l'issue de l'étape de séchage.

10. Procédé d'élimination d'une molécule acide d'un flux d'hydrocarbures contenant au moins une molécule acide, dans lequel le flux d'hydrocarbures est mis en contact durant une étape d'adsorption avec un adsorbant selon les revendications 1 à 5, ladite étape d'adsorption étant mise en oeuvre à une température entre -50 et 100°C, à une pression absolue comprise entre 0,01 MPa et 5 MPa et à une vitesse volumique horaire comprise entre 50 et 50000 h⁻¹.

11. Procédé d'élimination selon la revendication 10, dans lequel la molécule acide est le COS et/ou le CO₂.

12. Procédé d'élimination selon les revendications 10 ou 11, dans lequel on effectue une étape de régénération de l'adsorbant une fois que l'adsorbant est au moins partiellement saturé en molécules acides.

13. Procédé d'élimination selon la revendication 12, dans lequel l'étape de régénération est effectuée par la mise en contact de l'adsorbant au moins partiellement saturé en molécules acides avec un gaz ou un liquide à une température comprise entre 20 et 500°C, à une pression absolue comprise entre 0,01 MPa et 5 MPa et à une vitesse volumique horaire comprise entre 50 et 50000 h⁻¹.

## Patentansprüche

1. Adsorbens, umfassend einen Aluminiumoxidträger und mindestens ein alkalisches Element, wobei das Adsorbens durch Einleitung mindestens eines alkalischen Elements, das mit Natrium identisch oder von diesem unterschieden ist, auf einen Aluminiumoxidträger erhalten wird, dessen Natriumgehalt, ausgedrückt in Na₂O Äquivalent, vor der Einleitung des oder der alkalischen Elemente zwischen 1500 und 3500 ppm Gewicht bezogen auf das Gesamtgewicht des Trägers beträgt.

2. Adsorbens nach Anspruch 2, bei dem das alkalische Element unter Natrium und Kalium ausgewählt ist.

3. Adsorbens nach den Ansprüchen 1 oder 2, bei dem der Gehalt an alkalischem Element bezogen auf das Gesamtgewicht des Adsorbens zwischen 1 und 60 Gew.-% des Elements beträgt.

4. Adsorbens nach einem der Ansprüche 1 bis 3, bei dem der Aluminiumoxidträger vor der Einleitung des oder der alkalischen Elemente Gesamtporenvolumen zwischen 0,3 und 1 cm³.g⁻¹ und eine spezifische Oberfläche zwischen 50 und 450 m².g⁻¹ hat.

5. Adsorbens nach einem der Ansprüche 1 bis 4, das von Kalium und dem Aluminiumoxidträger gebildet ist, der einen Natriumgehalt, ausgedrückt in Na₂O Äquivalent, vor Einleitung des Kaliums zwischen 1500 und 3500 ppm Gewicht bezogen auf das Gesamtgewicht des Trägers hat.

6. Verfahren zur Herstellung des Adsorbens, wie in einem der Ansprüche 1 bis 5 definiert, umfassend die folgenden Schritte:
a) Herstellen einer wässrigen Lösung, die mindestens eine Alkali-Vorstufe enthält,
b) Imprägnieren eines Aluminiumoxidträgers mit einem Natriumgehalt, ausgedrückt in Na₂O Äquivalent, zwischen 1500 und 3500 ppm Gewicht bezogen auf das Gesamtgewicht des Trägers mit der nach Schritt a) erhaltenen wässrigen Lösung,
c) Reifenlassen des imprägnierten Trägers aus Schritt b) in einem mit Wasser gesättigten geschlossenen Raum,
d) Trocknen des Feststoffs aus Schritt c).

7. Verfahren zur Herstellung des Adsorbens, wie in einem der Ansprüche 1 bis 5 definiert, umfassend die folgenden Schritte:
a') Mischen eines Aluminiumoxidträgers mit einem Natriumgehalt, ausgedrückt in Na₂O Äquivalent, zwischen 1500 und 3500 ppm Gewicht bezogen auf das Gesamtgewicht des Trägers mit einem Pulver mindestens einer Alkali-Vorstufe,
b') eventuell Zerkleinern des nach Schritt a') erhaltenen Gemisches auf eine Korngröße zwischen 2 und 100 µm,
c') Formen des Gemisches aus Schritt b') im Beisein von Wasser, um ein Material zu erhalten,
d') Trocknen des geformten Materials aus Schritt c').

8. Verfahren zur Herstellung des Adsorbens, wie in einem der Ansprüche 1 bis 5 definiert, umfassend die folgenden Schritte:
a") Mischen eines Aluminiumoxidträgers mit einem Natriumgehalt, ausgedrückt in Na₂O Äquivalent, zwischen 1500 und 3500 ppm Gewicht bezogen auf das Gesamtgewicht des Trägers mit einer Lösung, umfassend mindestens eine Alkali-Vorstufe, um eine Paste zu erhalten,
b") Formen der in Schritt a") erhaltenen Paste,
c") Trocknen der geformten Paste aus Schritt b").

9. Verfahren nach den Ansprüchen 6 bis 8, bei dem ein Kalzinierungsschritt bei einer Temperatur zwischen 280 und 550 °C unter Luft bei trockener oder feuchter Atmosphäre ach dem Trocknungsschritt durchgeführt wird.

10. Verfahren zur Entfernung eines sauren Moleküls aus einem Kohlenwasserstoffstrom, der mindestens ein saures Molekül enthält, bei dem der Kohlenwasserstoffstrom während eines Adsorptionsschritts mit einem Adsorbens nach den Ansprüchen 1 bis 5 in Kontakt gebracht wird, wobei der Adsorptionsschritt bei einer Temperatur zwischen - 50 und 100 °C, bei einem absoluten Druck zwischen 0,01 MPa und 5 MPa und bei einer stündlichen Volumengeschwindigkeit zwischen 50 und 50000 h⁻¹ eingesetzt wird.

11. Entfernungsverfahren nach Anspruch 10, bei dem das saure Molekül COS und/oder CO₂ ist.

12. Entfernungsverfahren nach den Ansprüchen 10 oder 11, bei dem ein Schritt der Regeneration des Adsorbens durchgeführt wird, wenn das Adsorbens zumindest teilweise mit sauren Molekülen gesättigt ist.

13. Entfernungsverfahren nach Anspruch 12, bei dem der Regenerationsschritt durch Inkontaktbringen des zumindest teilweise mit sauren Molekülen gesättigten Adsorbens mit einem Gas oder einer Flüssigkeit bei einer Temperatur zwischen 20 und 500 °C, bei einem absoluten Druck zwischen 0,01 MPa und 5 MPa und bei einer stündlichen Volumengeschwindigkeit zwischen 50 und 50000 h⁻¹ durchgeführt wird.

## Claims

1. Adsorbent comprising an alumina support and at least one alkali element, said adsorbent being obtained by the introduction of at least one alkali element, identical to or different from sodium, onto an alumina support the sodium content of which, expressed as Na₂O equivalent, before the introduction of the alkali element or elements, is comprised between 1500 and 3500 ppm by weight with respect to the total weight of the support.

2. Adsorbent according to claim 2, in which the alkali element is selected from sodium and potassium.

3. Adsorbent according to claims 1 or 2, in which the content of alkali element with respect to the total weight of the adsorbent is comprised between 1 and 60% by weight of said element.

4. Adsorbent according to one of claims 1 to 3, in which the alumina support before the introduction of the alkali element or elements has a total pore volume comprised between 0.3 and 1 cm³.g⁻¹ and a specific surface area between 50 and 450 m².g⁻¹.

5. Adsorbent according to one of claims 1 to 4, which is constituted by potassium and the alumina support having a sodium content, expressed as Na₂O equivalent, before the introduction of the potassium, comprised between 1500 and 3500 ppm by weight with respect to the total weight of the support.

6. Process for the preparation of the adsorbent as defined in one of claims 1 to 5, comprising the following steps:
a) preparing an aqueous solution containing at least one alkali precursor,
b) impregnating an alumina support having a sodium content, expressed as Na₂O equivalent, comprised between 1500 and 3500 ppm by weight with respect to the total weight of the support, with the aqueous solution obtained at the end of step a),
c) leaving the impregnated support originating from step b) to mature in a water-saturated closed vessel,
d) drying the solid originating from step c).

7. Process for the preparation of the adsorbent as defined in one of claims 1 to 5, comprising the following steps:
a') mixing an alumina support having a sodium content, expressed as Na₂O equivalent, comprised between 1500 and 3500 ppm by weight with respect to the total weight of the support, with a powder of at least one alkali precursor,
b') optionally grinding the mixture obtained at the end of step a') to a granulometry comprised between 2 and 100 µm,
c') forming the mixture originating from step b') in the presence of water so as to obtain a material,
d') drying the formed material originating from step c').

8. Process for the preparation of the adsorbent as defined in one of claims 1 to 5, comprising the following steps:
a") mixing an alumina support having a sodium content, expressed as Na₂O equivalent, comprised between 1500 and 3500 ppm by weight with respect to the total weight of the support, with a solution comprising at least one alkali precursor, in order to obtain a paste,
b") forming the paste obtained in step a"),
c") drying the formed paste originating from step b").

9. Process according to claims 6 to 8, in which a calcination step is carried out at a temperature comprised between 280 and 550°C in air in a dry or humid atmosphere at the end of the drying step.

10. Process for the elimination of an acidic molecule from a hydrocarbon flow containing at least one acidic molecule, in which the hydrocarbon flow is brought into contact during an adsorption step with an adsorbent according to claims 1 to 5, said adsorption step being carried out at a temperature between -50 and 100°C, at an absolute pressure comprised between 0.01 MPa and 5 MPa and at an hourly space velocity comprised between 50 and 50000 h⁻¹.

11. Elimination process according to claim 10, in which the acidic molecule is COS and/or CO₂.

12. Elimination process according to claims 10 or 11, in which a step of adsorbent regeneration is carried out once the adsorbent is at least partially saturated with acidic molecules.

13. Elimination process according to claim 12, in which the regeneration step is carried out by bringing the adsorbent, at least partially saturated with acidic molecules, into contact with a gas or a liquid at a temperature comprised between 20 and 500°C, at an absolute pressure comprised between 0.01 MPa and 5 MPa and at an hourly space velocity comprised between 50 and 50,000 h⁻¹.
